# EUROPEAN PATENT SPECIFICATION

(11) **EP 0 557 535 B1**
(45) Date of publication and mention of the grant of the patent: **17.12.1997**
(21) Application number: 92920009.5
(22) Date of filing: 16.09.1992
(51) Int. Cl.: C12P 21/08, C12N 5/28, C12N 15/08, A61K 39/395

(54) **HUMAN MONOCLONAL ANTIBODY AGAINST GLYCOPROTEIN IIb/IIIa**
HUMANER MONOKLONALER ANTIKÖRPER GEGEN GLYCOPROTEIN-IIB/IIIA
ANTICORPS MONOCLONAL HUMAIN CONTRE LA GLYCOPROTEINE IIb/IIIa

(30) Priority: 17.09.1991 JP 262640/91
(43) Date of publication of application: 01.09.1993
(73) Proprietor: TEIJIN LIMITED, Osaka-shi, Osaka 541 (JP)
(72) Inventor: MATSUMOTO, Yoh-ichi, Musashino-shi Tokyo 180 (JP); IKEDA, Yasuo, Toshima-ku Tokyo 170 (JP); HANDA, Makoto, Shibuya-ku Tokyo 150 (JP); KAWAMURA, Takashi, Hino-shi Tokyo 191 (JP); MURAKAMI, Toshinobu, Hino-shi Tokyo 191 (JP); SASAKI, Satoshi, Hachioji-shi Tokyo 192 (JP); KIMURA, Tsuyoshi, Tucson, AZ (US); SAGAWA, Tomoko, Hino-shi Tokyo 191 (JP)
(74) Representative: Harrison, Ivor Stanley
(86) International application number: JP9201181
(87) International publication number: WO9306232

(56) References cited:
- WO-A-90/06133
- WO-A-90/06134
- WO-A-90/11783
- THROMBOSIS RESEARCH, vol. 38, no. 5, 1985; D. HEINRICH et al., pp. 547-560/
- BLOOD, vol. 68, no. 3, 1986; B.S. COLLER et al., pp. 783-786/
- BLOOD, vol. 70, no. 1, 1987; D.J. NUGENT et al., pp. 16-22/
- CANCER & CHEMOTHERAPY, vol. 10, 1983; N. TOMOKI et al., pp. 2575-2581/

## Description

The present invention relates to human monoclonal antibody (hereinafter abbreviated as MCA), to human glycoprotein IIb/IIIa (GPIIb/IIIa) and a hybridoma producing the same.

It has been clarified that platelets play an important role in the generation and progress of thrombosis, and interest has been shown in anti-platelet therapy as one type of anti-thrombus therapy. When endothelial cells of a blood vessel are peeled off by any cause, platelets having receptors for von Willebrand factor (hereinafter abbreviated as vWF) adhere to the endothelium, are activated, and cause a release reaction and aggregation resulting in platelet thrombus formation. It is known that in platelet thrombus formation, receptors on platelets for vWF in adhesion are glycoprotein Ib(GPIb), and the aggregation of platelets is caused by binding glycoprotein IIb/IIIa(GPIIb/IIIa) molecules to each other via fibrinogen(Fbg) or vWF. The binding sites thereof have also been studied.

To date, several anti-platelet agents which inhibit platelet functions, such as adhesion, aggregation, and release reactions, and experimental thrombus formation have been developed. However, because many of these have a platelet aggregation inhibitory action as an aspect of their pharmacological activity, they have low specificity and involve side effects. For example, aspirin, which is also a well-known analgesic and antipyretic agent, causes side effects such as haemorrhage in the digestive tract, and inhibition of synthesis of prostacyclin in the endothelium of a blood vessel. Moreover, in the case of Ticlopidine, side effects such as symptoms of stomach, intestine and liver disorders, or leucocyte diseases, etc., are observed.

Recently, a technique for preparing mouse MCA has been established and the use of an anti-platelet mouse MCA as an anti-platelet agent has been attempted. For example, H. K. Gold et al., [J. Clin. Invest. 86, 651-659 (1990)], prepared mouse MCA which was specific to human GPIIb/IIIa and inhibited platelet aggregation, and used the same in a clinical trial. However, although it provided an inhibitory action to platelet aggregation, it is not believed that the mouse MCA is satisfactory for use in treatment or prophylaxis in humans due to problems of half-life time and immunogenicity.

Therefore, MCA of human origin, rather than mouse origin, is essential for safe application to humans for treatment and prophylaxis of thrombosis. Generally, MCA of human origin is produced from a hybridoma obtained by fusion of a human B lymphocyte capable of producing an antibody which is specific to GPIIb/IIIa and inhibits platelet aggregation, and an established lymphocyte cell line such as myeloma cell line; or is produced from lymphoblastoid cells obtained by transformation of said human B lymphocytes with EB virus.

From around 1980 to the present time, a great deal of research into the preparation of human MCA has been carried out. However, both of the above-mentioned techniques involve intrinsic difficulties, and there is no established technique for obtaining human MCA, such as there is for mouse MCA. However, if human B lymphocytes could be efficiently immunized, and a hybridoma capable of producing human MCA specific to human GPIIb/IIIa and having an ability to inhibit platelet aggregation could be obtained, MCA could be obtained therefrom in high yield and would be desirable as a medicament from the point of view of safety.

Since 1985, mouse monoclonal antibodies which specifically bind to GPIIb/IIIa and inhibit aggregation of human platelets have been reported in several papers (for example, B. S. Coller et al., Blood, Vol.68, No. 3, 1986, 783-786).

Moreover, a human monoclonal antibody which specifically binds to GPIIb/IIIa is described by D.J. Nugent et al., [Blood, Vol. 70, No. 1, 1987, 16-22; and in WO 90/06134], but there is no evidence that such antibody is able to inhibit aggregation of human platelets. There is also no reference in the cited prior art to any connection between inhibition of fibrinogen binding with GPIIb/IIIa and inhibition of platelet aggregation, nor on human MCA able to inhibit the binding of fibrinogen with GPIIb/IIIa.

Accordingly, the present invention provides a human monoclonal antibody able to specifically bind to human glycoprotein IIb/IIIa, to inhibit aggregation of human platelets and to inhibit the binding of fibrinogen to GPIIb/IIIa, or active fragments thereof.

The present invention also relates to a hybridoma constructed by fusion of human lymphocytes and mouse myeloma cells, which produces said monoclonal antibody, or progeny thereof.

The present invention further relates to a process for production of said monoclonal antibody characterized by culturing said hybridoma.

The present invention still further relates to a process for production of hybridoma characterized by selecting a hybridoma producing said monoclonal antibody.

In the accompanying drawings:

Figure 1 is a graph showing binding of human monoclonal antibodies of the present invention to GPIIb/IIIa in the presence or absence of Ca⁺⁺. In the graph, (a) shows binding of ICF2C8, and (b) shows binding of 1AD2-1.

Figure 2 is a graph showing the effect of the present human monoclonal antibodies on binding of Fbg and GPIIb/IIIa.

Figure 3 is a graph showing the effect of the present human monoclonal antibodies on binding of vWF and GPIIb/IIIa.

Figure 4 is a chart of flow cytometry showing binding of ICF2C8 to GPIIb/IIIa on the surface of platelets. In the chart, (a) shows the case wherein no antibody reaction was allowed, (b) shows the case wherein anti-GPIIb/IIIa human MCA reaction was not allowed and only FITC-labeled anti-human IgM goat antibody reacted to platelets, and (c) shows the case wherein after reaction of ICF2C8, FITC-labeled anti-human IgM goat antibody reacted.

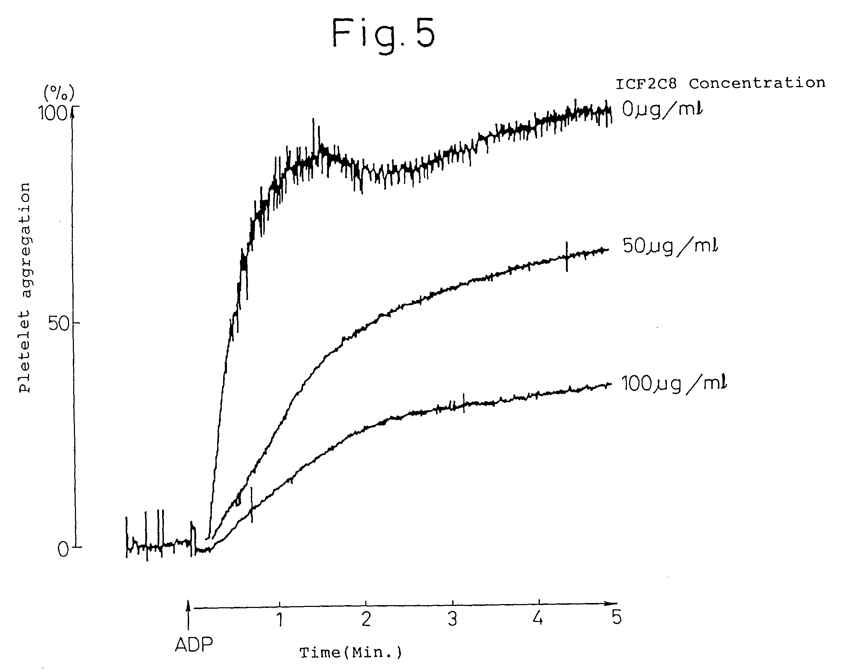

Figure 5 is a chart showing platelet aggregation inhibitory activity of ICF2C8.

Human lymphocytes used in the present invention are obtained from the spleen, lymph node, peripheral blood, bone marrow, tonsils and adenoids, of idiopathic thrombocytopenic purpura (ITP) patients. For the purpose of the present invention, although lymphocytes from any materials can be used, the lymph node, spleen or tonsils from an ITP patient are preferably used.

As the mouse myeloma cell lines, 8-azaguanine resistant cell lines are advantageously used, including known P3x65Ag8, P3-NSl/l-Ag4-1, P3x63AgU1, SP2/OAgl4, P363Ag8.6.5.3, MPC11-45.6, TG1.7, SP-1, etc., from a BALB/C mouse.

According to the present invention, prior to fusing human lymphocytes and mouse myeloma cells, T lymphocytes are preferably eliminated by treating human lymphocytes with a complement and mouse MCA to human lymphocytes (for example, OKT3 from Ortho Diagnostic), or with AET-treated sheep erythrocytes and Ficoll (Trade Mark).

For preparing human MCA according to the present invention, for example, a human solid lymphoid tissue is removed from an ITP patient by operation, and the removed tissue is gently disrupted with scissors and a scalpel to prepare a cell suspension. T lymphocytes are eliminated from the cell suspension using one of the following procedures.
(1) The cell suspension is overlayed on a Ficoll-Paque (Registered Trade Mark) layer and centrifuged to separate lymphocytes. Next, the lymphocytes are treated with a half volume of a complement and anti-human T lymphocyte mouse MCA to destroy T lymphocytes, and the remaining B lymphocytes are collected by centrifugation.
(2) The cell suspension is mixed with AET-treated sheep erythrocytes, and B cells are separated by the Ficoll-Paque (Registered Trade Mark) centrifugation method.

By using these methods, the rate of generation of hybridoma is enhanced in comparison with the case where non-treated lymphocytes are used.

Human lymphocytes thus obtained are fused with mouse myeloma cells. For example, human lymphocytes and myeloma cells are mixed at a ratio of 10:1 - 1:100, preferably 1:1 - 1:10, and an appropriate cell fusion solution, for example, RPMI 1640 containing about 35% polyethylene glycol (molecular weight about 1,000 to 6,000) and about 7.5% dimethylsulfoxide is added thereto. The mixture is then stirred at a temperature between room temperature and 37°C for 1 to a few minutes, gradually diluted with RPMI 1640 containing 10% fetal calf serum and, after washing the cells, the concentration of the cells is adjusted to 1 x 10⁵ to 5 x 10⁵cells/ml with HAT (hypoxanthine-aminopterin-thymidine) selection medium.

Next, 0.2 ml each of this cell suspension is seeded in a 96-well plate, and cultured in air containing 5% CO₂ at 35°C to 38°C for 2 to 3 weeks.

Prior to seeding of the cell suspension, mouse peritoneal cells are seeded into the 96-well plate as a feeder cells layer, and the medium is removed immediately before the addition of the fused cells. In HAT medium, only hybridomas survive; 8-azaguanine-resistant myeloma cells and fusion cells from myeloma cells alone cannot survive (non-fused antibody-producing cells die in a few days).

After culturing, an antibody produced in the culture medium is tested for its ability to bind to GPIIb/IIIa and for its ability to inhibit Fbg·GPIIb/IIIa binding, to select a hybridoma producing a desired antibody. Next, the hybridoma is picked up and cloned by a limiting dilution method to establish a subclone stably producing MCA.

Mouse-human hybridoma thus obtained, producing antiGPIIb/IIIa human MCA of the present invention, may be frozen for storage. Such a hybridoma cell line and/or cell line derived therefrom can be cultured in a large amount, and a desired human MCA of the present invention can be obtained from the culture supernatant. Moreover, the hybridoma can be inoculated into an animal to generate a tumor, and human MCA can be obtained from the ascites or serum of the animal.

Among the present anti-GPIIb/IIIa human MCA obtained as described above, ICF2C8 had the following properties.
(1) It was binding-positive in ELISA using immobilized GPIIb/IIIa from human platelets.
(2) It inhibited the *in vitro* binding of VWF and Fbg and GPIIb/IIIa.
(3) It inhibited the *in vitro* binding of VWF and GPIIb/IIIa.
(4) It inhibited aggregation of human platelets with ADP or collagen in an assay system using an aggregometer.
(5) The present human MCA, ICF2C8 was of Ig class M (IgM) and the light chain is λ.

The present human monoclonal antibody which specifically binds to GPIIb/IIIa and inhibits platelet aggregation is promising as an active ingredient of a platelet aggregation inhibitory agent for treatment and prophylaxis of thrombosis. However, for this application, not only a native monoclonal antibody, but also active fragments having specific binding ability, such as Fab, F(ab')₂, can be used. Accordingly, the present invention includes such active fragments of a monoclonal antibody. These fragments can be prepared according to conventional procedures such as described in, for example, Shin Seikagaku Jikken Koza (New Biochemistry Experimental Course), Tokyo Kagaku Dojin.

The present invention will now be explained in detail with reference to various Examples.

### Example 1

### (1) Cell fusion

(a) Preparation of lymphocytes
   A spleen, removed from an ITP patient by operation, was disrupted with scissors and a scalpel to small sections, and the cells were suspended in medium A (RPMIl64O-lO% fetal calf serum(FCS)-2mM glutamine, lmM sodium pyruvate-20 µg/ml L-serine-0.05 µ/ml human insulin-80 µg/ml gentamycin sulfate). This cell suspension was overlayed on a Ficoll-Paque (Registered Trade Mark) solution, and centrifuged at 1,500 rpm for 20 minutes. Cells collected on the Ficoll-Paque (Registered Trade Mark) solution were removed, and washed once with phosphate-buffered saline (PBS) and twice with RPMI1640 by centrifugation, and finally suspended in PRM11640 to make 1 x 10⁷ cells/ml lymphocyte suspension.
(b) Cell fusion
   The lymphocytes were cultured in a 10% FCS-RPMI1640 medium containing 10% human B cell growth factor or 1% pokeweed mitogen for 3 to 4 days. Next, 3 x 10⁷ cells of the lymphocytes and mouse myeloma P3U1 cells (3 x 10⁷cells) were mixed in RPMI1640, and the cells were precipitated by centrifugation (1,600 rpm, 5 minutes). After removing the supernatant, the cell pellet was disrupted by tapping the tube, and 1 ml of a polyethylene glycol solution (35% V/V polyethylene glycol # 1000-7.5% V/V dimethyl sulfoxide-RPMI1640) was added thereto. The cell suspension was allowed to stand at room temperature for one minute.
   Next, to this suspension was added 2 ml of RPMI1640, and after allowing to stand for one minute an additional 2 ml of RPM11640 was added thereto. After allowing the suspension to stand for two minutes, 4 ml of HAT medium (95 µM hypoxanthine-0.4 µM aminopterin-16 µM thymidine in the medium A) was added thereto, and the cell suspension was allowed to stand for 2 minutes. Next, 8 ml of HAT medium was added to the cell suspension, which was then allowed to stand for 2 minutes. Next, 24 ml of HAT medium was added to the cell suspension, which was then allowed to stand at 37°C for 30 minutes. Finally the suspension was made up to 75 - 150 ml with HAT medium.
   About 200 µl each of this cell suspension was seeded into a 96-well flat culture plate. Note, prior to the seeding, 2 x 10⁴ cells/well of ICR mouse (male) peritoneal cells were inoculated onto the plate, and the culture medium was removed immediately before the inoculation of the fused cells. The plate was incubated at 37°C in a CO₂-incubator. Half of the medium was replaced once a week with HT medium (HAT medium from which aminopterin had been omitted), and the culturing was continued until hybridoma clones appeared.

### (2) Screening

At the time at which hybridoma colonies appeared, binding activity to human GPIIb/IIIa and inhibitory activity to Fbg·GPIIb/IIIa binding were determined for each well by the following procedures (a) and (b), and hybridomas of positive colonies were cloned.
(a) Binding activity to human GPIIb/IIIa
   Human GPIIb/IIIa was diluted to 2 µg/ml with A buffer (1.27 mM Ca⁺⁺/0.9 mM Mg⁺⁺/PBS(pH 7.2)), and 50 µl/well of the dilution was put into a 96-well ELISA plate, which was then allowed to stand at 4°C overnight. After that, the GPIIb/IIIa solution was discarded, and 200 µl/well of 10% FCS/A buffer was put into the plate, which was then allowed to stand at 37°C for 120 minutes. After washing the plate with 0.002% Hibitane (Registered Trade Mark) three times, 50 µl/well of culture supernatant of the hybridoma was put into the plate to allow reaction at 37°C for 60 minutes.
   After washing the plate three times with 0·002% Hibitane (Registered Trade Mark), human MCA bound to GPIIb/IIIa was detected by adding 50 µl/well of alkaline phosphatase-labeled anti-human IgG diluted 10⁴ times with 10% FCS/A buffer, and reacting at 37°C for 60 minutes. After washing 5 times with 0.002% Hibitane (Registered Trade Mark), 100 µl/well of a solution prepared by dissolving 10 mg/ml of disodium p-nitrophosphate in 0.25 mM MgCl₂/1M diethanolamine (pH9.8) was added, and the color was developed to an appropriate level at room temperature. After that, the absorbance at 405 nm was measured to assess the binding activity to GPIIb/IIIa.
(b) Inhibitory activity to Fbg.GPIIb/IIIa binding
   Human fibrinogen (Fbg) was diluted to 10 µg/ml with A buffer, and 100 µl/well of the dilution was put into a 96-well ELISA plate, which was then allowed to stand at 37°C for 60 minutes. After that, the wells were washed with 1% BSA/1% FCS/Hanks' solution. 200 µl/well of 10% FCS/0.1% NaN₃/A buffer was put into the wells of the plate, which was then allowed to stand at 37°C for 60 minutes, and washed three times with 0.002% Hibitane. 0.25 µg/ml human GPIIb/IIIa diluted with 10% FCS/A buffer and the same volume of a hybridoma supernatant were mixed to allow a reaction at 37°C for 60 minutes, and 50 µl/well of the reaction mixture was added in to the plate for binding at 37°C for 60 minutes.
   After washing three times with 0.002% Hibitane (Registered Trade Mark), 50 µl/well of anti-GPIIb/IIIa mouse MCA (Serotec, MCA468) diluted with 10% FCS/A buffer to 500 ng/ml, was added and reacted at 37°C for 60 minutes to measure the amount of bound GPIIb/IIIa. After washing three times with 0.002% Hibitane (Registered Trade Mark), 50 µl/well of peroxidase-labeled anti-mouse IgG antibody (TAGO) diluted 10⁴ times with 10% FCS/A buffer was added to allow reaction at 37°C for 60 minutes.
   Next, the plate was washed 5 times with 0.002% Hibitane. 0.45 mg/ml TMBZ-HCl (pH 2.0) was mixed with the same volume of 0.017% H₂O₂/59 mMNaHPO₄/41 mM citric acid (pH 4.3), and 100 µl/well of the mixture was added to develop the color to an appropriate level at room temperature. 100 µl/well of IM H₂SO₄ was added to stop color development, and absorbance at 450 nm was measured to determine the amount of GPIIb/IIIa which bound to Fbg so as to assess inhibitory activity to Fbg·GPIIb/IIIa binding.

### (3) Cloning

The hybridomas which showed a positive reaction in the screening (2) were cloned as follows. First, 2 x 10⁴ cells/well of mouse peritoneal cells were inoculated into a 96-well plate. Next, the medium was removed 1 hour to one day later, and 10 cells/well of hybridoma was seeded into the 96 well plate. HT medium was used for the first cloning, and medium A was used for the second cloning. After culturing for 2 to 3 weeks, antibody activity was measured and positive clones were picked up.

### (4) Experimental result

Cell fusion and screening were repeated 80 times using (about 10 kinds of) lymphocytes obtained from the spleens of ITP patients. Although about 10% of the hybridomas were positive in binding activity to GPIIb/IIIa in the screening system (a) of the above paragraph (2), among them only one hybridoma had inhibitory activity to Fbg·GPIIb/IIIa binding in the screening system (b) of the above paragraph (2).

The hybridoma which was positive in both of the screening systems was cloned successfully to establish hybridoma ICF2C8 which stably produces MCA having the above mentioned properties (Table 1). In addition, 7 hybridomas which do not show inhibitory activity to Fbg.GPIIb/IIIa binding but bind to GPIIb/IIIa were cloned successfully to establish hybridomas stably producing MCA (Table 1).

The hybridoma cell line ICF2C8 thus obtained was deposited at the Fermentation Research Institute Agency of Industrial Science and Technology, 1-3, Higashi 1-chome, Tsukuba-shi, Ibaraki-ken, Japan, as FERM BP-3596 on October 8, 1991, under the Budapest Treaty.

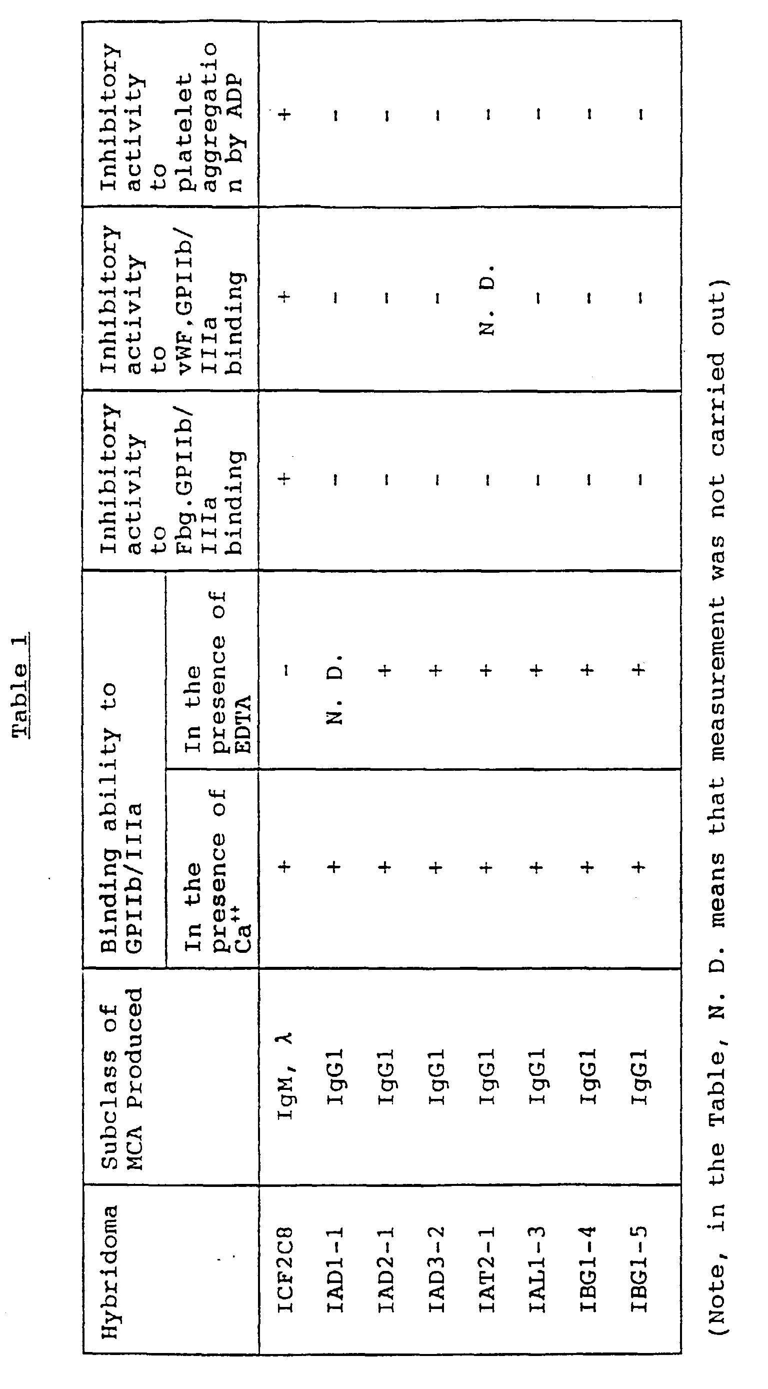

### Example 2

Hybridoma ICF2C8 was cultured in ITES/PVP medium to obtain a culture broth (3L) which was then used as a starting material. The culture broth was concentrated to about 500 ml by using an Amicon (Registered Trade Mark) YM30 membrane, and polyethylene glycol was added thereto to 10% of the final concentration, and the mixture was allowed to stand at 4°C for 120 minutes. The precipitate was collected by centrifugation and dissolved in 1M NaCl/20 mM phosphate buffer (pH 7.2) and applied to Con A Sepharose (Registered Trade Mark) equilibrated with the same buffer. This Con A Sepharose (Registered Trade Mark) was sequentially washed with 1M NaCl/20 mM NAPi (pH 7.2) and 1M NaCl/50 MmM AcNa (ph 4.0), and MCA was eluted with 0.5 λ-methyl mannose/0.5 M NaCl/20 mM NaPi (ph 7.2).

This eluted fraction was concentrated by using an Amicon (Registered Trade Mark) YM10 membrane and applied to Sepharose (Registered Trade Mark) CL-6B equilibrated with PBS, and a fraction containing IgM was recovered as ICF2C8 (5 mg). The ICF2C8 obtained by this method was confirmed to be IgM with at least 90% purity as measured by sodium dodecyl sulfate-polyacrylamide gel electrophoresis (SDS-PAGE).

### Example 3 Binding of human MCA to GPIIb/IIIa in presence or absence of Ca⁺⁺

### (1) Binding of human MCA to GPIIb/IIIa in the presence of Ca⁺⁺

Purified GPIIb/IIIa was diluted to 1 µg/ml with A buffer (1.27 mM Ca⁺⁺/0.9 mM Mg⁺⁺/PBS (pH 7.2)), and 100 µl/well of the dilution was put into a 96-well ELISA plate, which was then incubated at 4°C overnight. The GPIIb/IIIa solution was discarded and 175 µl/well of 2% BSA/A buffer was added. After incubation at 37°C for 120 minutes, the plate was washed three times with A buffer, and 100 µl/well of anti-GPIIb/IIIa human MCA (ICF2C8 or IAD2-1) diluted with 0.2% BSA/A buffer to 0 - 5 µg/ml was added thereto to allow reaction at 37°C for 60 minutes.

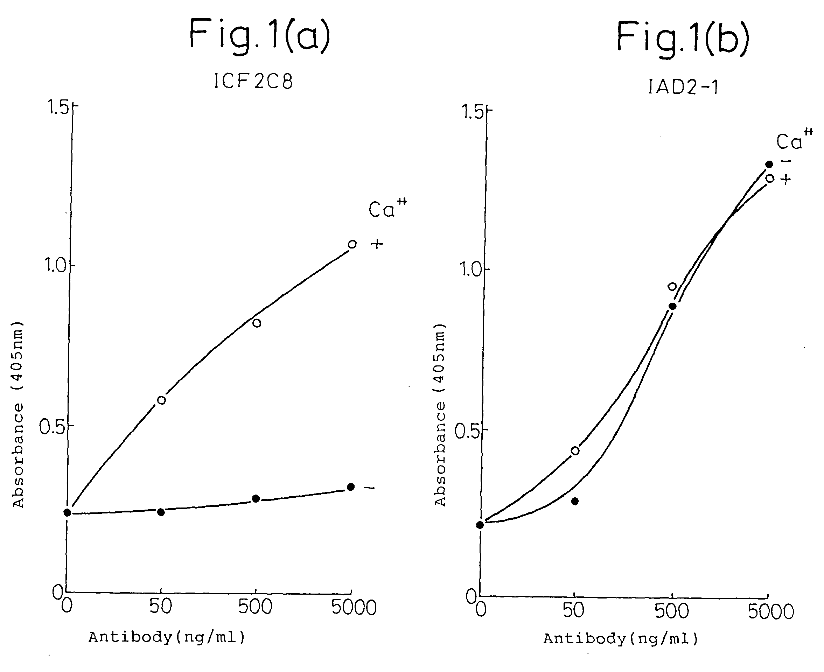

After washing three times with A buffer, the amount of human MCA bound to GPIIb/IIIa was measured by adding 100 µl/well of alkaline phosphatase-labeled anti-human IgM (or anti-human IgG) antibody diluted 104 fold with 0.2% BSA/A buffer and reacted at 37°C for 60 minutes. After washing the plate four times with A buffer, 100 µl/well of solution prepared by dissolving 10 mg/ml of disodium p-nitrophosphate in 0.25 mM MgCl₂/1M diethanolamine (pH 9.8) was added thereto, and color was developed at room temperature to an appropriate level. After that, absorbance at 405 nm was measured and from the absorbance the binding ability of human MCA to GPIIb/IIIa was assessed. The results are shown in Fig. 1.

### (2) Binding of human MCA to GPIIb/IIIa in the absence of Ca⁺⁺

Purified GPIIb/IIIa was diluted to 1 µg/ml with A buffer, and 100 µl/well of the dilution was added to the plate, which was then incubated at 4°C overnight. The GPIIb/IIIa solution was discarded, and 175 µl/well of 2% BSA/B buffer (10 mM EDTA/PBS) (pH 7.2)) was added to the plate, which was then incubated at 37°C for 120 minutes. After washing the plate three times with B buffer, 100 µl/well of anti-GPIIb/IIIa human MCA (ICF2C8 or IAD2-1) diluted to 0 - 5 µg/ml with 0.2% BSA/B buffer was added thereto to react at 37°C for 60 minutes.

After washing the plate three times with A buffer, the amount of human MCA bound to GPIIb/IIIa was measured by adding 100 µl/well of alkaline phosphatase-labeled anti-human IgM (or anti-human IgG) antibody diluted 10⁴ fold with 0.2% BSA/A buffer to react at 37°C for 60 minutes. After washing the plate four times with A buffer, the same color development operation as that in the presence of Ca⁺⁺ was carried out to assess the binding ability of MCA to GPIIb/IIIa. The results are shown in Fig. 1.

According to Fig. 1(a), in case of ICF2C8, absorbance increased in a dose dependent manner only in the presence of Ca⁺⁺, while it is not increased in the absence of Ca⁺⁺. Accordingly, it was found that ICF2C8 binds to GPIIb/IIIa only in the presence of Ca⁺⁺, and it does not bind to GPIIb/IIIa in the absence of Ca⁺⁺.

In addition, according to Fig. 1(b), it was found that IAD2-1 binds to GPIIb/IIIa regardless of the presence or absence of Ca^{*++}.

### Example 4 Effect of human MCA on binding of Fbg and GPIIb/IIIa

Human fibrinogen (Fbg) was diluted to 10 µg/ml with A buffer, and 100 µl/well of the dilution was added to a 96-well ELISA plate, which was then incubated at 37°C for 60 minutes. After that the wells were washed with 1% BSA/1% FCS/Hanks' solution. 200 µl/well of 10% FCS/0.1% NaN₃/A buffer was added and, after incubation at 37°C for 60 minutes, the plate was washed three times with 0.002% Hibitane (Registered Trade Mark). 0.25 µg/ml of human GPIIb/IIIa diluted with 10% FCS/A buffer was mixed with the same volume of anti-human GPIIb/IIIa human MCA (ICF2C8 or IAD2-1) having one of various concentrations and the mixture was reacted at 37°C for 60 minutes. 50 µl/well of the reaction mixture was added in the ELISA plate to bind at 37°C for 60 mintues.

After washing the plate three times with 0.002% Hibitane (Registered Trade Mark), the amount of bound GPIIb/IIIa was measured by adding 50 µl/well of anti-GPIIb/IIIa mouse MCA (Serotec, MCA468) diluted with 10% FCS/A buffer to 500 ng/ml to react at 37°C for 60 minutes. After washing the plate three times with 0.002% Hibitane (Registered Trade Mark), 50 µl/well of peroxidase-labeled anti-mouse IgG antibody (TAGO) diluted 10⁴ fold with 10% FCS/A buffer was added thereto to react at 37°C for 60 minutes. After that, the plate was washed 5 times with 0.002% Hibitane (Registered Trade Mark).

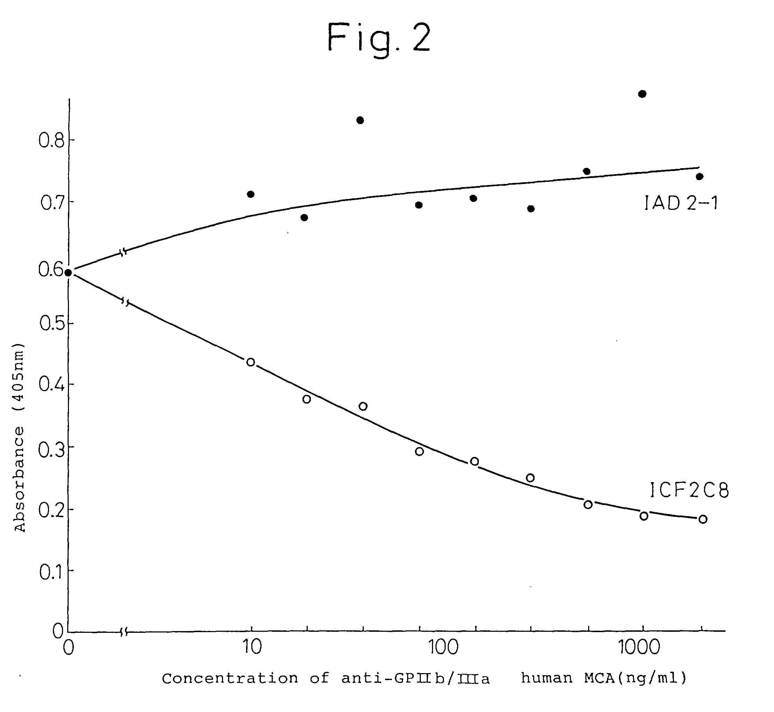

0.45 mg/ml TMBZ-HCl (pH 2.0) was mixed with the same volume of 0.017% H₂O₂/59 mM Na₂HPO₄/41 mM citric acid (pH 4.3), and 100 µl/well of the mixture was added to develop the color to an appropriate level at room temperature. 100 µ/well of 1M H₂SO₄ was added to terminate the color development, and the absorbance at 450 nm was measured to determine the amount of GPIIb/IIIa bound to Fbg. The results are shown in Fig. 2.

It is found in Fig. 2 that ICF2C8 decreases absorbance in a dose-dependent manner. Namely, binding of Fbg and GPIIb/IIIa is inhibited by ICF2C8 in a dose-dependent manner, wherein ICF2C8 at a final concentration of 2.5 µg/ml inhibited 70% of the binding. However, IAD2-1, belonging to anti-GPIIb/IIIa human MCA, did not inhibit binding of Fbg and GPIIb/IIIa.

### Example 5 Effect of ICF2C8 on binding of vWF and GPIIb/IIIa

To a 96-well ELiSA plate was added 100 µl/well of anti-vWF polyclonal antibody diluted to 21.6 µg/ml with A buffer, and the plate was incubated at 4°C overnight. The antibody solution was discarded, and 175 µl/well of 2% BSA/A buffer was added to the plate, which was then incubated at 37°C for 120 minutes. The wells were washed three times with A buffer, and 100 µl/well of von Willebrand disease-treating agent Hemate (registered trade mark) P (Hoechst Japan) diluted to 0.625 units/ml with 0.2% BSA/A buffer was added to the plate. The plate was incubated at 37°C for 120 minutes to bind vWF.

After washing the wells three times with A buffer, GPIIb/IIIa(2 µg/ml) diluted with 0.2% BSA/A buffer was mixed with the same volume of anti-GPIIb/IIIa human MCA (0 to 10 µg/ml), and the mixture was reacted at 37°C for 90 minutes. 100 µl/well of the mixture was added thereto to react at 37°C for 90 minutes for binding of vWF and GPIIb/IIIa. After washing the wells three times with A buffer, GPIIb/IIIa bound to vWF was measured by adding 100 µl/well of anti-GPIIb/IIIa mouse MCA (Serotec, MCA 468) diluted to 500 ng/ml with 0.2% BSA/A buffer to react at 37°C for 60 minutes.

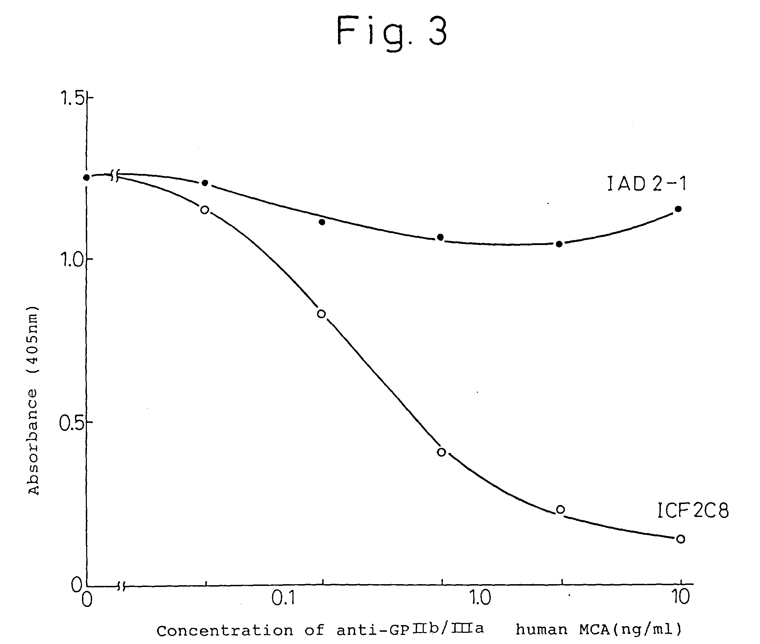

After washing the plate three times with A buffer, 100 µl/well of alkaline phosphatase-labeled anti-mouse IgG antibody (TAGO Inc. Cat Nr. 6550) diluted 10⁴ fold with 0.2% BSA/A buffer was added thereto to react at 37°C for 60 minutes. After washing four times with A buffer, 100 µl/well of a solution prepared by dissolving 10 mg/ml of disodium p-nitrophosphate in 0.25 mM MgCl₂/1M diethanolamine (pH 9.8) was added to develop the color at room temperature for 60 minutes. After that absorbance at 405 nm was measured. From this absorbance, the amount of binding of vWF and GPIIb/IIIa was assessed. The relationship between the concentration of anti-GPIIb/IIIa human MCA added and the absorbance at 405 nm obtained as above was shown in Fig. 3.

According to Fig. 3, an anti-GPIIb/IIIa human MCA, ICF2C8, decreased absorbance in a dose-dependent manner, wherein at 10 µg/ml it decreased absorbance to 11%. Namely, ICF2C8, at a concentration of 10 µg/ml, inhibited 89% of binding of vWF and GPIIb/IIIa. An anti-GPIIb/IIIa human MCA, 1AD2-1 does not inhibit binding of vWF and GPIIb/IIIa

### Example 6 Binding Ability of ICF2C8 to platelet surface

Fresh human blood and 3.8% U/V sodium citrate were mixed at a ratio of 9:1, and the mixture was centrifuged at 1000 rpm for 10 minutes to obtain a platelet rich plasma. This platelet rich plasma was mixed with anti-GPIIb/IIIa human monoclonal antibody (ICF2C8) to make a final concentration of 5 µg/ml monoclonal antibody, and the mixture was reacted at room temperature for 30 minutes. The reaction mixture was centrifuged (2,500 rpm, 10 minutes), and the precipitated platelets was washed once with buffer C (15 mM Tris-HCl/150 mM NaCl (pH 7.4)).

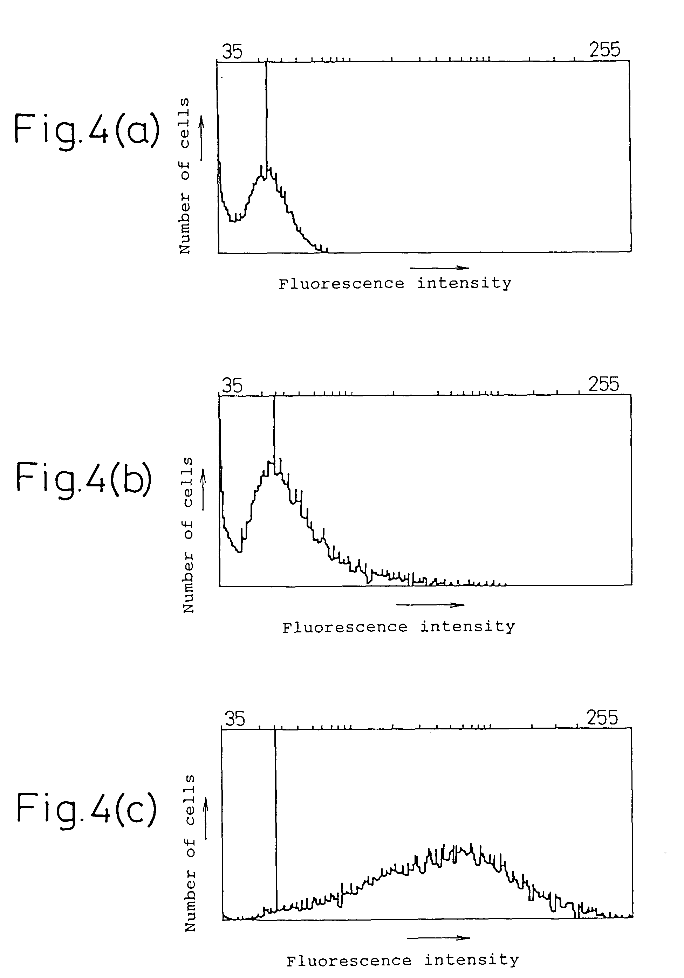

The platelets were suspended in buffer C, and reaction with FITC-labeled anti-human IgM goat antibody was carried out at room temperature for 30 minutes. This reaction mixture was centrifuged (2500 rpm, 10 minutes), and precipitated platelets were washed once with buffer A. The platelets were resuspended in buffer A and subjected to flow cytometry. The results are shown in Fig. 4.

In Fig. 4, (a) is a chart of flow cytometry for the case wherein no antibody reaction was allowed, (b) is the case wherein no anti-GPIIb/IIIa human monoclonal antibody reaction was allowed, but only FITC-labeled anti-human IgM goat antibody reacted with platelets, and (c) is the case wherein ICF2C8 reacted and then FITC-labeled anti-human IgM goat antibody reacted.

From Fig. 4, it is clear that the FITC-labeled anti-human IgM goat antibody does not bind to platelet surfaces under the present conditions since peak positions of (a) and (b) are not different. Where ICF2C8 reacted with platelets, the position of the peak of (c) moved to the right in comparison with the peaks of (a) and (b). This means that ICF2C8 reacts not only with purified GPIIb/IIIa but also with GPIIb/IIIa on the surface of platelets.

### Example 7 Inhibitory Activity of ICF2C8 to platelet aggregation

Fresh human blood and 3.8% u/v sodium citrate were mixed at a ratio of 9:1, and the mixture was centrifuged at 1000 rpm for 10 minutes to obtain a platelet rich plasma (PRP). 450 µl of the PRP was reacted with 45 µl of different concentrations of ICF2C8 at 37°C for one minute and ADP was added thereto to a final concentration of 4.8 µM ADP to cause platelet aggregation, which was observed at 37°C for 5 minutes by a aggregometer. The results are shown in Fig. 6.

According to Fig. 6, ICF2C8 inhibits platelet aggregation in a dose-dependent manner, wherein at a concentration of 100 µg/ml it inhibited the platelet aggregation by 70%.

The present monoclonal antibodies and active fragments thereof are promising for use as active ingredients for platelet aggregation inhibitory agents for prophylactis or treatment of thrombus.

Reference to deposited microorganisms under rule 13-2 and depository authority:
Fermentation Research Institute Agency of Industrial Science and Technology; 1-3, Higashi 1-chome, Tsukubashi Ibaraki-ken
ICF2C8 FERM BP-3596
Deposition Date: October 8, 1991

## Claims

1. A human monoclonal antibody having the ability to specifically bind to human glycoprotein IIb/IIIa, to inhibit aggregation of human platelets and to inhibit the binding of fibrinogen to GPIIb/IIIa, or active fragments thereof.

2. A hybridoma obtainable by fusion of human lymphocytes and mouse myeloma cells and producing a human monoclonal antibody according to Claim 1, or progeny thereof.

3. A process for production of a human monoclonal antibody having the ability to specifically bind to human glycoprotein IIb/IIIa to inhibit aggregation of human platelets and to inhibit the binding of fibrinogen to GPIIb/IIIa, characterized by culturing a hybridoma according to claim 2.

4. A process according to Claim 3, characterized in that human lymphocytes and mouse myeloma cells are fused and a hybridoma producing said monoclonal antibody is selected.

5. A platelet aggregation inhibitory agent comprising a human monoclonal antibody according to Claim 1.

## Patentansprüche

1. Menschlicher monoklonaler Antikörper mit der Fähigkeit, spezifisch an menschliches Glycoprotein IIb/IIIa zu binden, um die Aggregation von menschlichen Plättchen zu verhindern und die Bindung von Fibrinogen an GPIIb/IIIa oder aktiven Fragmenten hiervon zu verhindern.

2. Hybridom, erhältlich durch Fusion eines menschlichen Lymphozyten und einer Maus-Myelomzelle, welches einen menschlichen monoklonalen Antikörper gemäß Anspruch 1 produziert, oder ein Abkömmling hiervon.

3. Verfahren zur Herstellung eines menschlichen monoklonalen Antikörpers, welcher die Fähigkeit aufweist, spezifisch an menschliches Glycoprotein IIb/IIIa zu binden, um die Aggregation von menschlichen Plättchen zu verhindern und die Bindung von Fibrinogen an GPIIb/IIIa zu verhindern, gekennzeichnet durch das Kultivieren eines Hybridoms gemäß Anspruch 2.

4. Verfahren nach Anspruch 3, gekennzeichnet dadurch, daß man menschliche Lymphozyten und Maus-Myelomzellen verschmilzt und ein Hybridom, welches den monoklonalen Antikörper produziert, auswählt.

5. Mittel zur Verhinderung der Plättchenaggregation, umfassend einen menschlichen monoklonalen Antikörper gemäß Anspruch 1.

## Revendications

1. Anticorps monoclonal humain ayant la capacité de se lier spécifiquement à la glycoprotéine IIb/IIIa humaine, d'inhiber l'agrégation des plaquettes humaines et d'inhiber la liaison du fibrinogène à GPIIb/IIIa, ou ses fragments actifs.

2. Hybridome susceptible d'être obtenu par fusion de lymphocytes humains et de cellules de myélome de souris et produisant un anticorps monoclonal humain selon la revendication 1, ou sa descendance.

3. Procédé de production d'un anticorps monoclonal humain ayant la capacité de se lier spécifiquement à la glycoprotéine IIb/IIIa humaine, d'inhiber l'agrégation des plaquettes humaines et d'inhiber la liaison du fibrinogène à la GPIIb/IIIa, caractérisé par la culture d'un hybridome selon la revendication 2.

4. Procédé selon la revendication 3, caractérisé en ce qu'on fusionne des lymphocytes humains et des cellules de myélome de souris et qu'on sélectionne un hybridome produisant ledit anticorps monoclonal.

5. Antiagrégant plaquettaire comprenant un anticorps monoclonal humain selon la revendication 1.
